# EUROPÄISCHE PATENTANMELDUNG

(11) **EP 1 559 395 A2**
(43) Veröffentlichungstag der Anmeldung: **03.08.2005**
(21) Anmeldenummer: 05000096.7
(22) Anmeldetag: 05.01.2005
(51) Int. Cl.: A61K 7/06, A61K 7/48, B01F 17/00

(54) **Mikroemulsion**

(30) Priorität: 29.01.2004 DE 102004004394
(71) Anmelder: Wella Aktiengesellschaft, 64274 Darmstadt (DE)
(72) Erfinder: Maillefer, Sara, Dr., 1749 Middes (CH); Chambettaz, Daniel, 1717 St. Ursen (CH); Franzke, Michael, Dr., 64380 Rossdorf (DE)

(57) **Zusammenfassung**

Es wird eine vorzugsweise optisch klare, transparente oder durchscheinende Mikroemulsion beschrieben mit einem Gehalt an 20 bis 60 Gew.% Wasser, 3 bis 20 Gew.% einer Ölphase, welche im wesentlichen aus bei 25 °C flüssigen, hydrophoben Ölen besteht, 20 bis 60 Gew.% einer Emulgatormischung, welche im wesentlichen besteht aus mindestens einem ersten Emulgator, der ausgewählt ist aus hydrierten und nicht hydrierten ethoxylierten Rizinusölen und mindestens einem zweiten Emulgator, welcher einen NRU₅₀-Wert von größer 110 µg/ml aufweist. Die Mikroemulsion ist als kosmetisches, dermatologisches oder pharmazeutisches Mittel anwendbar, insbesondere als Mittel zur Haarbehandlung.

## Beschreibung

Gegenstand der Erfindung ist eine Mikroemulsion, welche insbesondere als kosmetisches, dermatologisches oder pharmazeutisches Mittel zur Haut- oder Haarbehandlung anwendbar ist, vorzugsweise in Form eines optisch klaren, transparenten oder zumindest durchscheinenden Produktes vorliegt und einen Gehalt an Wasser, einer Ölphase aus flüssigen, hydrophoben Ölen sowie einer Emulgatormischung aus mindestens zwei bestimmten, ausgewählten Emulgatoren aufweist.

Mikroemulsionen sind makroskopisch homogene, optisch transparente, niedrigviskose, thermodynamisch stabile Mischungen aus zwei miteinander nicht mischbaren Flüssigkeiten und mindestens einem nichtionischen oder einem ionischen Tensid, das zwei hydrophobe Reste enthält. Wird den beiden ineinander unlöslichen Komponenten, z.B. Wasser und ein unpolarer Kohlenwasserstoff, ein ionisches Tensid mit nur einem hydrophoben Rest zugesetzt, benötigt man zur Ausbildung der Mikroemulsion noch ein Cotensid, meist einen kurzkettigen aliphatischen Alkohol. Bei einer Mikroemulsion handelt es sich um eine ternäre Mischung aus Wasser, hydrophober Phase und Tensidphase. Mit dem Transmissionselektronenmikroskop läßt sich zeigen, dass Mikroemulsionen in submikroskopisch stark fluktuierende, bikontinuierliche Öl- und Wasser-Domänen strukturiert sind, wobei die Größe der Domänen zwischen 3 und 100 nm durch die Grenzflächenspannung zwischen der wasserreichen und der ölreichen Phase in Gegenwart der gesättigten monomolekularen Tensid-Schicht beständig ist. Häufig sind Mikroemulsionen fest oder halbfest und zeigen ein als "Ringing-Effekt" bekanntes Verhalten. Viele pharmazeutische und kosmetische Zubereitungen stellen Mikroemulsionen dar. Ein generelles Problem der Mikroemulsionen besteht darin, dass sie im Vergleich zu normalen, aus den gleichen, an sich unbedenklichen Inhaltsstoffen aufgebauten (Makro-)Emulsionen, ein erhöhtes Risiko für Hautunverträglichkeiten, insbesondere Haut- und Augenreizungen aufweisen. Bei Verwendung von besonders milden und hautfreundlichen Tensiden sellt sich das Problem von nicht ausreichender Stabilität und es kann zu Trübungen, Auftrennungen oder farblichen Veränderungen kommen oder die gewünschte Härte oder Festigkeit des Produktes läßt mit der Zeit nach. Es bestand daher die Aufgabe, eine Mikroemulsion zur Verfügung zu stellen, welche besonders haut- und/oder augenverträglich ist, eine ausreichende Langzeitstabilität aufweist und eine gewünschte Klarheit und Festigkeit aufweist, die auch über einen längeren Zeitraum hinweg nicht wesentlich beeinträchtigt wird.

Es wurde nun gefunden, dass die Aufgabe gelöst wird durch eine Mikroemulsion der nachfolgend beschriebenen Zusammensetzung. Gegenstand der Erfindung ist eine vorzugsweise optisch klare, transparente oder durchscheinende Mikroemulsion mit einem Gehalt an
(A) 20 bis 60 Gew.%, vorzugsweise 30 bis 55 Gew.% Wasser,
(B) 3 bis 20 Gew.% einer Ölphase, im wesentlichen bestehend aus bei 25 °C flüssigen, hydrophoben Ölen, wobei die Ölphase darin gelöste, lipophile Stoffe enthalten kann,
(C) 20 bis 60 Gew.% einer Emulgatormischung, im wesentlichen bestehend aus
   (c1) mindestens einem ersten Emulgator, ausgewählt aus ethoxylierten Rizinusölen und ethoxylierten hydrierten Rizinusölen und
   (c2) mindestens einem zweiten Emulgator, welcher einen NRU₅₀-Wert von größer 110 µg/ml aufweist und welcher vorzugsweise einen NRU₅₀-Wert aufweit, der größer ist als derjenige von mit 40 mol Ethylenoxid ethoxyliertem hydrierten Rizinusöl.

### Wässrige Phase (A)

In der wässrigen Phase können neben Wasser gegebenenfalls ein oder mehrere hydrophile Co-Lösungsmittel enthalten sein, z.B. kosmetisch verträgliche einwertige oder mehrwertige Alkohole. Geeignete einwertige Alkohole sind insbesondere solche mit 1 bis 4 C-Atomen wie z.B. Ethanol oder Isopropanol. Geeignete mehrwertige Alkohole sind insbesondere solche mit 2 bis 6 C-Atomen wie z.B. Ethylenglykol oder Propylenglykol, Glycerin aber auch Sorbitol, von denen Glycerin besonders bevorzugt ist. Die Co-Lösungsmittel werden vorzugsweise in einer Menge von 0 bis 15 Gew.% oder von 1 bis 10 Gew.% eingesetzt. In der wässrigen Phase können zusätzlich darin gelöste, hydrophile Wirk- und Zusatzstoffe enthalten sein.

### Ölphase (B)

Der Gehalt der Ölphase beträgt 3 bis 20 Gew.%, vorzugsweise 5 bis 15 Gew.%. Die Ölphase besteht im wesentlichen aus bei Raumtemperatur (25°C) flüssigen Komponenten sowie gegebenenfalls darin gelösten lipophilen Wirk- und Zusatzstoffen. Bei Raumtemperatur feste Wachse sind entweder nicht, oder nur in einer die optische Klarheit nicht beeinträchtigenden Menge, insbesondere in Mengen kleiner 5 Gew.%, vorzugsweise kleiner 1 Gew.% enthalten.

Flüssige Öle sind z.B. pflanzliche Öle, tierische Öle, Mineralöle, Silikonöle, Kohlenwasserstofföle, hydrierte Polyolefine, flüssige Alkohole mit mindestens 8 C-Atomen, insbesondere verzweigte Alkohole wie z.B. Guerbetalkohole, Öle aus Fettsäuren und Polyolen, Öle aus Fettsäuren und einwertigen C1- bis C30-, vorzugsweise C3- bis C22-Alkoholen oder Mischungen der genannten Öle. Geeignete Öle sind insbesondere Cycloparaffine, Paraffinöle, Polydecen, Mineralöl, Isohexadekan, Dodekan, Isoeicosan, flüssige Polydimethylsiloxane, Cyclotetrasiloxan, Cyclopentasiloxan, Phenyltrimethicone, Isocetylpalmitat, Isopropylmyristat, Isopropylpalmitat, Isopropylstearat, Octylisostearat, Octylcocoat, Octylpalmitat, Octyldodecylmyristat, Caprylic/Capric Triglyceride, Butyloctanol, Hexyloctanol, Butyldecanol, Hexyldecanol, Octyldodecanol, Hexyldecanol, Stearylheptanoat, Isohexyldecanoat, Isodecyloctanoat, Dibutyladipate, Dicaprylylether, C12-15-Alkylbenzoate, hydriertes Polyisobuten, Squalan, Squalen, native Öle wie Jojobaöl, Olivenöl, Sonnenblumenöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macademianußöl, Maiskeimöl, Avocadoöl und ähnliche.

### Emulgatorphase (C)

Der Gehalt an Emulgatoren beträgt 20 bis 60 Gew.%, vorzugsweise 30 bis 50 Gew.%. Das Gewichtsverhältnis von Emulgator (c1) zu Emulgator (c2) beträgt vorzugsweise von 1:1 bis 6:1, insbesondere von 2:1 bis 4:1. Die Emulgatorphase besteht im wesentlichen aus den oben genannten Emulgatortypen (c1) und (c2). Insbesondere sind Emulgatoren mit NRU₅₀-Werten kleiner 110 µg/ml entweder nicht, oder nur in solchen geringen Mengen enthalten, dass der NRU₅₀-Wert der erfindungsgemäßen Mikroemulsion größer als 110 µg/ml, vorzugsweise größer als 200 µg/ml oder größer als 750 µg/ml ist. Insbesondere sind Emulgatoren mit NRU₅₀-Werten kleiner 110 µg/ml wie z.B. ethoxylierte Fettalkohole oder ethoxylierte Fettalkoholphosphatester nicht oder nur in Mengen kleiner 10 Gew.%, kleiner 5 Gew.% oder kleiner 1 Gew.% enthalten.

Zur Herstellung von Mikroemulsionen werden relativ hohe Emulgatormengen benötigt. Wegen des hohen Emulgatorgehalts haben herkömmliche Mikroemulsionen ein hohes Augenreizpotential. Es hat sich herausgestellt, dass der als Neutral Red Uptake-Test (NRU-Test) bekannte in vitro-Test gut geeignet ist zur Charakterisierung der Augenverträglichkeit von Mikroemulsionen und dass NRU₅₀-Werte geeignet sind zur Identifizierung von geeigneten Emulgatoren zur Herstellung von augenfreundlicheren Mikroemulsionen.

Der NRU-Test, beschrieben in E. Borenfreund, J. A. Puerner, Toxicology Letters 24 (1985), Seiten 119-124, ist ein mit Zellkulturen durchgeführter Zytotoxizitäts-Test. Bei Verwendung von Zellen der humanen HaCaT-Keratinocytzellinie anstelle der im oben genannten Artikel verwendeten tierischen Balb/c 3T3 Fibroblasten-Zellkulturen, erhält man bessere Voraussagen bezüglich des Augenreizpotentials.

NRU-Werte im Sinne der Erfindung sind NRU₅₀-Werte und bezeichnen diejenige Konzentration eines Stoffes oder einer Zusammensetzung, bei welcher die Aufnahme des Farbstoffs Neutralrot in menschliche Keratinocytzellen um 50% reduziert wird. Neutralrot (3-Amino-7-dimethylamino-2-methyl-phenazin Hydrochlorid) ist ein schwach kationischer Farbstoff, der leicht aus einem äußeren Medium durch die Zellmembran gesunder Zellen hindurch in das Zellinnere diffundiert. Änderungen der Zelloberfläche oder der Zellmembran durch potentielle Schadstoffe verringern die Farbstoffaufnahme und die Retention des Farbstoffs in der Zelle. Ein hoher NRU₅₀-Wert bedeutet eine geringe zellschädigende Wirkung und eine niedrige Zytotoxizität und ist ein Hinweis auf eine bessere Augenverträglichkeit der untersuchten Substanz bzw. Zusammensetzung.

### Emulgator (c1) auf Basis von Rizinusöl

Der erste Emulgator ist vorzugsweise in einer Menge von 10 bis 50 Gew.%, insbesondere von 15 bis 45 Gew.% enthalten. Der erste Emulgator ist ausgewählt aus ethoxylierten Rizinusölen und ethoxylierten hydrierten Rizinusölen. Der Ethoxylierungsgrad kann von 10 bis 100, insbesondere von 20 bis 60 betragen. Geeignet sind insbesondere Stoffe mit den INCI-Bezeichnungen PEG-x Castor Oil und PEG-x Hydrogenated Castor Oil, wobei x den Ethoxylierungsgrad angibt, z.B. hydriertes PEG-25 Rizinusöl, hydriertes PEG-35 Rizinusöl, hydriertes PEG-40 Rizinusöl, hydriertes PEG-45 Rizinusöl, hydriertes PEG-54 Rizinusöl oder hydriertes PEG-60 Rizinusöl.

### Co-Emulgator (c2)

Der zweite Emulgator ist vorzugsweise in einer Menge von 3 bis 30 Gew.%, insbesondere von 5 bis 20 Gew.% enthalten. Der zweite Emulgator ist vorzugsweise ausgewählt aus Fettsäurezuckerestern und ethoxylierten Fettsäuremonoglyceriden, wobei die Fettsäuren vorzugweise 8 bis 30, insbesondere 12 bis 22 C-Atome aufweisen. Der Zuckerbaustein der Fettsäurezuckerester ist z.B. Saccharose. Fettsäurezuckerester sind z.B. solche mit den INCI-Bezeichnungen Sucrose Cocoate, Sucrose Dilaurate, Sucrose Distearate, Sucrose Laurate, Sucrose Myristate, Sucrose Oleat, Sucrose Palmitate, Sucrose Ricinoleate, Sucrose Stearat.

Ethoxylierte Fettsäuremonoglyceride sind z.B. solche der allgemeinen Formel CₙH₍₂ₙ₊₁₎-CO-OCHCH(-OH)CH₂(-OCH₂CH₂-)ₘOH, wobei n eine ganze Zahl ist, die vorzugweise von 7 bis 29, insbesondere von 11 bis 21 beträgt und m eine Zahl ist, die den Ethoxylierungsgrad angibt, welcher z.B. von 2 bis 100, insbesondere von 3 bis 40 oder von 5 bis 12 beträgt. Fettsäuremonoglyceride sind z.B. solche mit den INCI-Bezeichnungen PEG-x Glyceryl Cocoate, PEG-x Glyceryl Isostearate, PEG-x Glyceryl Laurate, PEG-x Glyceryl Oleate, PEG-x Glyceryl Ricinoleate, PEG-x Glyceryl Sesquioleate, PEG-x Glyceryl Stearate, PEG-x Glyceryl Talowate, wobei x den Ethoxylierungsgrad angibt.

### Wirkstoffe

In einer bevorzugten Ausführungsform enthält die erfindungsgemäße Mikroemulsion zusätzlich mindestens einen Wirkstoff, welcher ausgewählt sein kann aus haarkosmetischen, hautkosmetischen, dermatologischen und/oder pharmazeutischen Wirkstoffen. Wirkstoffe sind z.B. mehrwertige Alkohole, UV-Filter, Lichtschutzmittel, Antioxidantien, Radikalfänger, Antischuppenwirkstoffe, Duftstoffe, Diagnostika, Therapeutika, Vitamine, rückfettende Stoffe, Pflanzen- und Kräuterextrakte, Protein- und Seidenhydrolysate, Haarglanzgeber, haarfestigende oder haarpflegende nichtionische, anionische, kationische, zwitterionische oder amphotere Polymere, wobei die Polymere synthetischen oder natürlichen Ursprungs sein können, sowie deren Kombination. Die Menge an Wirkstoff kann je nach Wirksamkeit und Anwendungszweck variieren, z.B. von 0,001 bis 10 Gew.%.

Das erfindungsgemäße Produkt kann darüber hinaus übliche Zusatzbestandteile enthalten, z.B. Konservierungsmittel, bakterizide und fungizide Wirkstoffe wie z.B. 2,4,4-Trichlor-2-hydroxydiphenylether, Parabene oder Methylchlorisothiazolinon, in einer Menge von etwa 0,01 bis 1,0 Gew.%; Puffersubstanzen, wie z.B. Natriumcitrat oder Natriumphosphat, in einer Menge von etwa 0,1 bis 1,0 Gew.%; Anfärbestoffe, wie z.B. Fluorescein Natriumsalz, in einer Menge von etwa 0,1 bis 1,0 Gew.%.

Eine bevorzugte Ausführungsform betrifft eine Mikroemulsion mit einem Gehalt an
(A) 20 bis 60 Gew.% Wasser,
(B) 3 bis 20 Gew.% einer Ölphase bestehend aus bei 25 °C flüssigen, hydrophoben Ölen, ausgewählt aus pflanzlichen Ölen, tierischen Ölen, Mineralölen, Silikonölen, Kohlenwasserstoffölen, Ölen aus Fettsäuren und Polyolen, Öle aus Fettsäuren und einwertigen C1- bis C30-Alkoholen oder Mischungen der genannten Öle, wobei die Ölphase darin gelöste, lipophile Stoffe enthalten kann und keine oder weniger als 5 Gew.% bei Raumtemperatur festen Wachse enthält,
(C) 20 bis 60 Gew.% einer Emulgatormischung, welche zu mindestens 90 Gew.%, bezogen auf die Emulgatormenge, besteht aus
   (c1) mindestens einem ersten Emulgator, ausgewählt aus ethoxylierten hydrierten Rizinusölen mit einem Ethoxylierungsgrad von 10 bis 80 und
   (c2) mindestens einem zweiten Emulgator, ausgewählt aus Fettsäurezuckerestern und ethoxylierten Fettsäuremonoglyceriden.

Die erfindunggemäße Mikroemulsion kann entsprechend ihrem Aufbau als kosmetisches Mittel, topisches dermatologisches Mittel oder pharmazeutisches Mittel verwendet werden, z.B. als Haarbehandlungsmittel, Haarstylingmittel, Haarpflegemittel, Hautcreme, Hautschutzcreme, Tagescreme, Nachtcreme, Sonnenschutzmittel für Haut oder Haar oder als Grundlage für pharmazeutische Zubereitungen.

### Anwendungsverfahren

Ein weiterer Gegenstand der Erfindung ist ein Verfahren zur Haarbehandlung, wobei ein eine erfindungsgemäße Mikroemulsion enthaltendes oder daraus bestehendes Haarbehandlungsmittel bereit gestellt wird, das Haarbehandlungsmittel auf das Haar aufgebracht wird und eine Frisur erstellt wird.

Die Anwendung kann auf trockenem oder leicht feuchtem Haar erfolgen. Die Einsatzmenge kann je nach Haarlänge und gewünschtem Effekt eine erbsen- bis haselnussgroße Menge sein, die vorteilhafterweise vor dem Aufbringen auf das Haar in den Handflächen verrieben wird. Durch die spezielle Konsistenz lässt sich das Produkt gezielt und ohne große Einarbeitung besonders leicht im Haar verteilen. Es gibt dem Haar Stand, Definition, Struktur und Halt. Die Haare haben ein angenehmes Aussehen und wirken insbesondere weder stumpf noch ölig glänzend. Das Produkt eignet sich ausgezeichnet zum kreativen Modellieren und Re-Modellieren moderner Stylings.

Das erfindungsgemäße Mittel zeichnet sich besonders durch seine Klarheit und Transparenz aus. Daher wird das Mittel vorteilhafterweise auch in eine optisch ansprechende Verpackung aus durchsichtigem oder durchscheinendem Material abgefüllt. Als Behältermaterial kommen insbesondere Glas und durchsichtige oder durchscheinende Kunststoffe wie z.B. Polyethylen, Polypropylen oder Polyethylenterephtalat in Betracht.

Die nachfolgenden Beispiele sollen den Gegenstand der Erfindung näher erläutern.

### Beispiele

Die Bestimmung von NRU-Werten basiert auf dem in E. Borenfreund, J. A. Puerner, Toxicology Letters 24 (1985), Seiten 119-124 beschriebenen, standardisierten Testverfahren und auf der standardisierten Durchführungsvorschrift, erarbeitet für die COLIPA International Validation Study on Alternatives to the Draize Rabbit Eye Irritation Test (Brantom et al. 1997) mit den folgenden Änderungen: Verwendung der humanen Keratinozyten-Zelllinie HaCaT anstelle der BALB/c 3T3 Mausfibroblasten und Behandlung in serumfreiem Kulturmedium.

Das von den den Testsubstanzen ausgesetzten Zellen aufgenommene Neutralrot (3-Amino-7-dimethylamino-2-methyl-phenazin Hydrochlorid) wird extrahiert und spektrophotometrisch bei 540 nm gemessen. Toxische Effekte bewirken eine Reduktion der Menge an aufgenommenem Neutralrot gegenüber gesunden, nicht behandelten Kontrollzellen. Die Konzentration einer Testsubstanz, die zu einer Verringerung der aufgenommenen und gebundenen Neutralrotmenge um 50% gegenüber den Kontrollzellen führt, ist der NRU₅₀-Wert.

Die HaCaT-Zellen werden gezüchtet in Dulbecco's modified Eagle's medium (DMEM), ergänzt mit 2 mM L-Glutamin, 10% (v/v) foetal calf serum (FCS), 100 IU/ml penicillin und 100 µg/ml streptomycin bei 37 °C in einer Atmosphäre mit 95% Luftfeuchtigkeit und 5% CO₂. Ein Zellvorrat wird in eingefrorenem Zustand (-196°C, flüssiger Stickstoff) aufbewahrt in gleichen Teilen von je 1-2 * 10⁶ Zellen/ml in DMEM mit 20% FCS und 10% Dimethylsulfoxid.

Zur Durchführung der NRU-Tests werden die Zellen zunächst 24 Stunden in dem Wachstumsmedium gezüchtet und dann der Testsubstanz ausgesetzt in serumfreiem Keratinozyt-Nährmedium (keratinocyte growth medium KGM), einer modifizierten MCDB 153 Formulierung, vom Hersteller (Clonetics) ergänzt mit 0,1 ng/ml epidermal growth factor, 5 µg/ml Insulin, 0,5 µg/ml Hydrocortison, 0,15 mM Ca⁺⁺, 0,4% v/v bovine pituitary extract (7,5 mg/ml) und Antibiotika.

In Vorversuchen wird mit 8 verschiedenen Konzentrationen der Testsubstanz ein ungefährer NRU(50)-Wert ermittelt. Auf dieser Basis werden für die endgültige Messung 8 geeignete Konzentrationen um diesen ungefähren Wert herum ausgewählt. Die Messung wird mit einem unabhängigen Ansatz mit gleichen Konzentrationen wiederholt. Als Referenzsubstanz dient Natriumlaurylsulfat in Konzentrationen von 50, 25, 10 und 1 µg/ml.

Ungefähr 2 * 10⁴ HaCaT-Zellen in 250 µl Wachstumsmedium pro Vertiefung werden in den 60 zentralen Vertiefungen von Mikrotiterplatten mit je 96 Vertiefungen platziert. Die Eckvertiefungen dienen als Kontrolle und enthalten nur das Wachstumsmedium. Die Platten werden 24 Stunden bei 37°C in einer feuchten Atmosphäre mit 5%CO2/95% Luft inkubiert. Das Kulturmedium wird entfernt und entweder durch reines Behandlungsmedium oder durch das verschiedene Konzentrationen an der zu untersuchenden Testsubstanz oder der Referenzsubstanz enthaltende Behandlungsmedium ersetzt. Das Layout der Mikrotiterplatte sieht aus wie folgt:

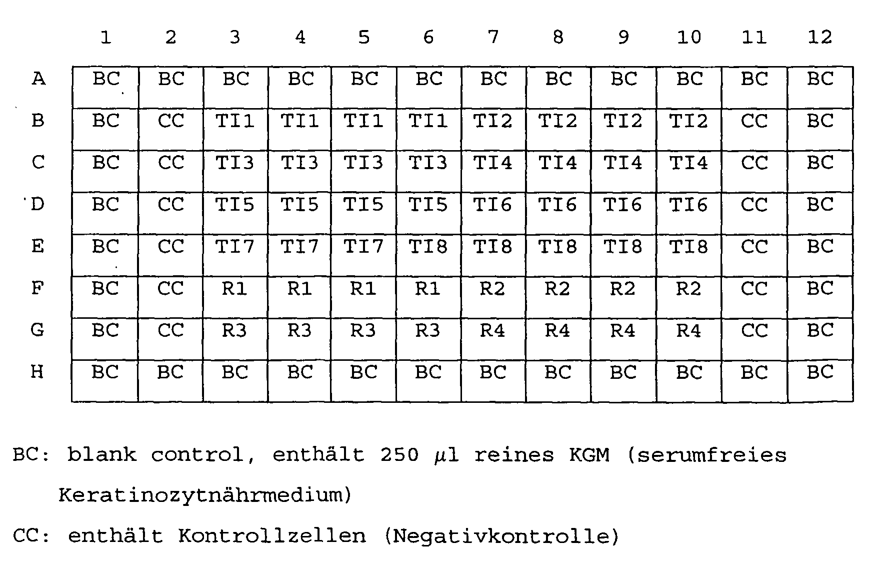

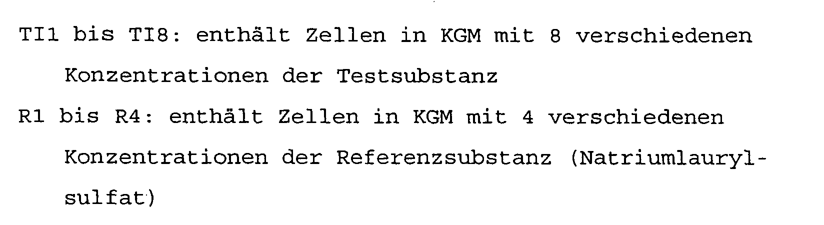

Nach einer Einwirkungsdauer von 24 Stunden wird das Behandlungsmedium durch Aufbewahrungsmedium ersetzt, welches 50 µg/ml Neutralrot enthält. Die Platten werden für 3 Stunden erneut in den Inkubator gestellt. Danach wird das Medium entfernt, die Zellen mit einem Fixativ (1% Formaldehyd, 1% Calciumchlorid) gewaschen und zur Farbstoffextraktion 100 µl einer Extraktionslösung (1% Eisessig in 50% Ethanol) pro Vertiefung zugefügt. Nach 20 Minuten wird mit einem Mikroplattenleser die Absorption bei 540 nm gemessen. Aus den Mittelwerten und nach Korrektur mit dem Nullwert aus den BC-Vertiefungen werden für die Testsubstanzen und für die Referenzsubstanz Konzentrations/Wirkungs-Diagramme aufgezeichnet, ausgedrückt als prozentuale Lebensfähigkeit der negativ Kontrollzellen (CC) gegenüber den jeweiliegen Testkonzentrationen. Der NRU₅₀-Wert, welcher diejenige Konzentration der Testsubstanz darstellt, bei der die Neutralrotaufnahme um 50% gegenüber der Negativkontrolle reduziert ist, wird durch Interpolation des erhaltenen Graphen des Konzentrations/Wirkungs-Diagramms bestimmt. Der erhaltene Wert ist akzeptabel, wenn der für die Referenzsubstanz Natriumlaurylsulfat erhaltene Wert im Bereich von 10 bis 30 µg/ml liegt.
Die im Folgenden angegebenen NRU-Werte sind nach der vorgenannten Methode bestimmte NRU₅₀-Werte in der Einheit µg/ml.

### Erfindungsgemäße Emulgatoren:

- Cremophor® RH 410:: PEG-40 Hydrogenated Castor Oil;
NRU₅₀ = 188 µg/ml
- Cremophor® CO60:: PEG-60 Hydrogenated Castor Oil;
NRU₅₀ = 203 µg/ml
- Cetiol® HE:: PEG-7 Glyceryl Cocoat; NRU₅₀ = 249 µg/ml
- Tegosoft® LSE 65K:: Sucrose Cocoat; NRU₅₀ = 198 µg/ml

### Nicht erfindungsgemäße Emulgatoren:

- Eumulgin® 05:: Oleth-5; NRU = 7,8
- Eumulgin® B2:: Ceteareth-20; NRU = 5,8
- Volpo® N20:: Oleth-20; NRU = 6,2

### Beispiel 1 (Mengenangaben in g)

| | A | B | C | D | E |
|---|---|---|---|---|---|
| PEG-40 Hydrogenated Castor Oil | 30 | 30 | 30 | 30 | 30 |
| PEG-7 Glyceryl Cocoat | 10 | 10 | 10 | 10 | 10 |
| Sucrose Cocoat | 10 | - | - | - | - |
| Octyldodecanol | - | - | - | - | 10 |
| Caprylic/Capric Triglycerid | 10 | 5 | 3,3 | 10 | - |
| Glycerin | - | 5 | 3,3 | - | - |
| Propylenglykol | - | - | 3,3 | - | - |
| Panthenol | 0,1 | 0,1 | 0,1 | 0,1 | 0,1 |
| Konservierungsmittel, UV-Filter, Parfüm | q.s. | q.s. | q.s. | q.s. | q.s. |
| Wasser | Ad 100 | Ad 100 | Ad 100 | Ad 100 | Ad 100 |

| NRU-Werte: | | | | | |
|---|---|---|---|---|---|
| | A | B | C | D | E |
| NRU₅₀ [µg/ml] | 1334 | 1184 | 976 | 2216 | 256 |

### Vergleichsbeispiele

| | F | G | H | I | J |
|---|---|---|---|---|---|
| PEG-40 Hydrogenated Castor Oil | - | 20 | 20 | 10 | 10 |
| PEG-60 Hydrogenated Castor Oil | 20 | - | - | - | - |
| Sucrose Cocoat | 10 | 10 | 10 | 10 | 10 |
| PEG-7 Glyceryl Cocoat | - | - | - | 10 | - |
| Ceteareth-20 | 10 | - | 10 | - | - |
| Oleth-5 | - | 10 | - | - | 10 |
| Oleth-20 | - | - | - | 10 | 10 |
| Octyldodecanol | - | - | 10 | 10 | - |
| Caprylic/Capric Triglycerid | 10 | 10 | - | - | 10 |
| Panthenol | 0,1 | 0,1 | 0,1 | 0,1 | 0,1 |
| Konservierungsmittel, UV-Filter, Parfüm | q.s. | q.s. | q.s. | q.s. | q.s. |
| Wasser | Ad 100 | Ad 100 | Ad 100 | Ad 100 | Ad 100 |

| NRU-Werte: | | | | | |
|---|---|---|---|---|---|
| | F | G | H | I | J |
| NRU₅₀ [µg/ml] | 51 | 83 | 53 | 30 | 36 |

| **Vergleichsbeispiele - Handelsprodukte** | |
|---|---|
| Produkt | NRU₅₀[µg/ml] |
| Tec ni art Digit Gloss (L'Oreal): | 22 |
| wässrige Phase: Wasser, Propylenglykol, Glycerin | |
| Ölphase: C12-15 Alkylbenzoat, Octyldodecylneopentanoate | |
| Emulgatoren: PEG-40 Hydrogenated Castor Oil, PPG5-Ceteth-20, Steareth-20, Oleth-3 Phosphat | |
| TRENDLINE gel wax normal (Goldwell): | 71 |
| wässrige Phase: Wasser, Sorbitol, Butylenglykol, Glycerin | |
| Ölphase: Paraffinum liquidum | |
| Emulgatoren: Oleth-5, Oleth-3 Phosphat, Ceteareth-20 | |
| Shape Up Soft Wax (Schwarzkopf) | 59 |
| wässrige Phase: Wasser, Sorbitol, Butylenglykol, Glycerin | |
| Ölphase: Paraffinum liquidum | |
| Emulgatoren: Oleth-5, Oleth-3 Phosphat | |

## Patentansprüche

1. Mikroemulsion mit einem Gehalt an
(A) 20 bis 60 Gew.% Wasser,
(B) 3 bis 20 Gew.% einer Ölphase, im wesentlichen bestehend aus bei 25 °C flüssigen, hydrophoben Ölen,
(C) 20 bis 60 Gew.% einer Emulgatormischung, im wesentlichen bestehend aus
(c1) mindestens einem ersten Emulgator, ausgewählt aus ethoxylierten Rizinusölen und ethoxylierten hydrierten Rizinusölen und
(c2) mindestens einem zweiten Emulgator, welcher einen NRU₅₀-Wert von größer 110 µg/ml aufweist.

2. Mikroemulsion nach Anspruch 1, **dadurch gekennzeichnet, dass** die hydrophoben Öle der Ölphase (B) ausgewählt sind aus pflanzlichen Ölen, tierischen Ölen, Mineralölen, Silikonölen, Kohlenwasserstoffölen, flüssigen Alkoholen mit mindestens 8 C-Atomen, Ölen aus Fettsäuren und Polyolen, Ölen aus Fettsäuren und einwertigen C1- bis C30-Alkoholen oder Mischungen der genannten Öle.

3. Mikroemulsion nach einem der vorhergehenden Ansprüche, **dadurch gekennzeichnet, dass** die Ölphase (B) keine bei Raumtemperatur festen Wachse in einer die optische Klarheit beeinträchtigenden Menge enthält.

4. Mikroemulsion nach einem der vorhergehenden Ansprüche, **dadurch gekennzeichnet, dass** der Ethoxylierungsgrad des Emulgators (c1) von 10 bis 100 beträgt.

5. Mikroemulsion nach einem der vorhergehenden Ansprüche, **dadurch gekennzeichnet, dass** der Emulgator (c1) ausgewählt ist aus hydriertem PEG-25 Rizinusöl, hydriertem PEG-35 Rizinusöl, hydriertem PEG-40 Rizinusöl, hydriertem PEG-45 Rizinusöl, hydriertem PEG-54 Rizinusöl und hydriertem PEG-60 Rizinusöl.

6. Mikroemulsion nach einem der vorhergehenden Ansprüche, **dadurch gekennzeichnet, dass** der Emulgator (c2) ausgewählt ist aus Fettsäurezuckerestern und ethoxylierten Fettsäuremonoglyceriden.

7. Mikroemulsion nach einem der vorhergehenden Ansprüche, **dadurch gekennzeichnet, dass** der Emulgator (c2) ausgewählt ist aus Saccharosecocoat und ethoxyliertem Glycerylcocoat.

8. Mikroemulsion nach einem der vorhergehenden Ansprüche, **dadurch gekennzeichnet, dass** das Gewichtsverhältnis von Emulgator (c1) zu Emulgator (c2) von 1: 1 bis 6:1 beträgt.

9. Mikroemulsion nach einem der vorhergehenden Ansprüche, **dadurch gekennzeichnet, dass** die Emulgatormischung (C) einen Emulgator mit einem NRU₅₀-Wert kleiner oder gleich 110 µg/ml nicht oder nur in einer Menge von kleiner 10 Gew.%, bezogen auf das Gesamtgewicht der Mikroemulsion, enthält.

10. Mikroemulsion nach einem der vorhergehenden Ansprüche, **dadurch gekennzeichnet, dass** sie zusätzlich mindestens einen haarkosmetischen, hautkosmetischen, dermatologischen und/oder pharmazeutischen Wirkstoff enthält.

11. Mikroemulsion nach Anspruch 10, **dadurch gekennzeichnet, dass** der Wirkstoff ausgewählt ist aus mehrwertigen Alkoholen, UV-Filtern, Lichtschutzmitteln, Antioxidantien, Radikalfängern, Antischuppenwirkstoffen, Duftstoffen, Diagnostika, Therapeutika, Vitaminen, rückfettenden Stoffen, Pflanzen- und Kräuterextrakten, Protein- und Seidenhydrolysaten, Haarglanzgebern, haarfestigenden oder haarpflegenden Polymeren sowie deren Kombination.

12. Mikroemulsion nach einem der vorhergehenden Ansprüche, **gekennzeichnet durch** einen Gehalt an
(A) 20 bis 60 Gew.% Wasser,
(B) 3 bis 20 Gew.% einer Ölphase bestehend aus bei 25 °C flüssigen, hydrophoben Ölen, ausgewählt aus pflanzlichen Ölen, tierischen Ölen, Mineralölen, Silikonölen, Kohlenwasserstoffölen, Ölen aus Fettsäuren und Polyolen, Ölen aus Fettsäuren und einwertigen C1- bis C30-Alkoholen oder Mischungen der genannten Öle, wobei die Ölphase darin gelöste, lipophile Stoffe enthalten kann und keine oder weniger als 5 Gew.% bei Raumtemperatur festen Wachse enthält,
(C) 20 bis 60 Gew.% einer Emulgatormischung, welche zu mindestens 90 Gew.%, bezogen auf die Emulgatormenge, besteht aus
(c1) mindestens einem ersten Emulgator, ausgewählt aus ethoxylierten hydrierten Rizinusölen mit einem Ethoxylierungsgrad von 10 bis 80 und
(c2) mindestens einem zweiten Emulgator, ausgewählt aus Fettsäurezuckerestern und ethoxylierten Fettsäuremonoglyceriden.

13. Mikroemulsion nach einem der vorhergehenden Ansprüche, **dadurch gekennzeichnet, dass** es in einer durchsichtigen oder durchscheinenden Verpackung vorliegt.

14. Verwendung einer Mikroemulsion nach einem der vorhergehenden Ansprüche zur Herstellung eines kosmetischen, dermatologischen oder pharmzeutischen Mittels.

15. Verwendung einer Mikroemulsion nach einem der Ansprüche 1 bis 13 zur Haarbehandlung.

16. Verfahren zur Haarbehandlung, wobei
- ein eine Mikroemulsion nach einem der Ansprüche 1 bis 13 enthaltendes oder daraus bestehendes Haarbehandlungsmittel bereit gestellt wird,
- das Haarbehandlungsmittel auf das Haar aufgebracht wird und
- eine Frisur erstellt wird.
